# EUROPÄISCHE PATENTSCHRIFT

(11) **EP 3 930 665 B1**
(45) Veröffentlichungstag und Bekanntmachung des Hinweises auf die Patenterteilung: **17.07.2024**
(21) Anmeldenummer: 20747379.4
(22) Anmeldetag: 27.07.2020
(51) Int. Cl.: A61K 8/06, A61K 8/37, A61K 8/46, A61K 8/73, A61Q 19/00

(54) **ACRYLAT- UND SILIKONFREIE KOSMETISCHE O/W-EMULSION**
ACRYLATE AND SILICON-FREE COSMETIC O/W EMULSION
ÉMULSION H/E COSMÉTIQUE EXEMPTE D'ACRYLATE ET DE SILICIUM

(30) Priorität: 28.08.2019 DE 102019212913
(43) Veröffentlichungstag der Anmeldung: 05.01.2022
(73) Patentinhaber: Beiersdorf AG, 22529 Hamburg (DE)
(72) Erfinder: SKUBSCH, Kerstin, 25497 Prisdorf (DE); FRANCK, Kerstin, 25451 Quickborn (DE); VOIGT, Nadine, 22111 Hamburg (DE); NEBEN, Jette Mareike, 22527 Hamburg (DE); WISCHHÖFER, Svea, 21109 Hamburg (DE); HERWIG, Katharina, 22529 Hamburg (DE)
(74) Vertreter: Beiersdorf AG
(86) Internationale Anmeldenummer: PCT/EP2020/071103
(87) Internationale Veröffentlichungsnummer: WO 2021/037455

(56) Entgegenhaltungen:
- EP-A1- 1 294 772
- EP-A1- 1 935 394
- EP-A1- 4 021 380
- EP-A1- 4 021 382
- WO-A1-2008/003685
- WO-A2-2007/017196

## Beschreibung

Kosmetische Produkte dienen im Allgemeinen nicht nur dazu schön und attraktiv auszusehen, sondern sie tragen mit ihrer Wirkung entscheidend zu einem gesteigerten Selbstwertgefühl und zum Wohlbefinden der Menschen bei. Dementsprechend werden die verschiedensten kosmetischen Produkte zur täglichen Reinigung und Pflege der menschlichen Haut eingesetzt.

Hautpflegeprodukte bestehen in der Regel aus Emulsionen. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Zur Stabilisierung und zur Verdickung von O/W-Emulsionen werden zumeist Acrylat-basiete Polymere in diese Formulierungen eingearbeitet. Acrylat-basierte Polymere sind Polymere, welche aus Homo- oder Copolymerisation mit Acryl- und/oder Methacrylsäure erhalten werden. Beispiele hierfür sind u.a. Carbomer oder Acrylate Copolymer.

Der Stand der Technik kennt unter anderem die Dokumente DE 10148825 A1, DE 19938756 A1 und DE 29924371 U1, welche pflegende O/W-Emulsionen mit Acrylat-basierten Polymeren offenbaren. Jedoch ist der Einsatz dieser Acrylat-basierten Polymere zunehmend in der Kritik, da deren biologische Abbaubarkeit nicht vollständig geklärt ist.

Ein weiterer oft verwendeter Bestandteil von O/W-Emulsionen zur Auftragung auf die Haut sind Silikonöle. Bei Auftragen einer O/W-Emulsion fühlt sich die Haut geschmeidig an. Zudem haben Silikonöle einen weichzeichnenden Effekt und wirken als optischer Faltenfiller.

Silikone sind per se für die Haut nicht schädlich. Als hautfremde Stoffe lösen sie keine Allergien oder Reizreaktionen aus. Einmal abgewaschen ist die Haut wieder wie vorher. Kritisch ist jedoch ebenfalls der Umstand dass die Umweltverträglichkeit von Silikonölen noch nicht abschließend beurteilt wurde bzw. sich die Substanzen in der Natur nur schwer abbauen. Folglich gilt es den Einsatz von Silikonölen in kosmetischen Zubereitungen zur Auftragung auf die Haut zu reduzieren.

EP1 935 394 offenbart eine acrylat- und silikonfreie kosmetische O/W-Emulsion enthaltend Natrium Cetearyl Sulfat und Glyceryl Stearate SE.

Problematisch ist jedoch der Umstand, dass O/W-Emulsionen die keine acrylathaltigen Polymere enthalten und bei denen auf den Einsatz von Silikonölen verzichtet wird oftmals dazu tendieren beim Verteilen auf der Haut weiße Rückstände zu hinterlassen.

Aufgabe der vorliegenden Erfindung war es daher eine acrylat- und silikonfreie kosmetische O/W-Emulsion bereitzustellen, welche die Nachteile des Standes der Technik nicht aufweist. Insbesondere war es Aufgabe eine derartige O/W-Emulsion bereitzustellen, welche beim Auftragen schnell einzieht und dabei geringere Mengen an Rückständen aufweist. Überraschend gelöst wird/werden die Aufgaben durch eine acrylat- und silikonfreie kosmetische O/W-Emulsion enthaltend
a) mindestens eine Substanz gewählt aus der Gruppe der Alkalimetallalkylsulfate, wobei die Alkylgruppe jeweils 15 bis 19 Kohlenstoffatome aufweist,
b) Hydroxypropylstärkephosphat, und
c) Glyceryl Stearate SE, gemäß den Ansprüchen.

Gegenstand der Erfindung ist auch die Verwendung eine Mischung aus
a) mindestens eine Substanz gewählt aus der Gruppe der Alkalimetallalkylsulfate, wobei die Alkylgruppe jeweils 15 bis 19 Kohlenstoffatome aufweist,
b) Hydroxypropylstärkephosphat, und
c) Glyceryl Stearate SE

in einer acrylat- und silikonfreien kosmetische O/W-Emulsion zur Reduzierung von weißen Rückständen beim Auftragen der Emulsion auf die Haut, gemäß den Ansprüchen.

Sollten nachfolgend Gewichtsprozentangaben (Gew.-%) ohne Bezugnahme auf eine bestimmte Zusammensetzung oder spezifische Mischung angegeben werden, so beziehen sich diese Angaben immer auf das Gesamtgewicht der kosmetischen O/W-Emulsion. Sollten nachfolgend Verhältnisse von Komponenten/Substanzen/Stoffgruppen offenbart werden, so beziehen sich diese Verhältnisse auf Gewichtsverhältnisse der genannten Komponenten/ Substanzen/Stoffgruppen.

Werden nachfolgend Gewichtsprozentbereiche für die Bestandteile der kosmetischen O/W-Emulsion angegeben, so umfasst die Offenbarung der vorliegenden Anmeldung ebenfalls alle Einzelwerte in Schritten von 0,1 Gew.-% innerhalb dieser Gewichtsprozentbereiche.

Die Formulierungen "erfindungsgemäß", "erfindungsgemäß vorteilhaft", "vorteilhaft im Sinne der Vorliegenden Erfindung" etc. beziehen sich im Rahmen der vorliegenden Offenbarung immer sowohl auf die erfindungsgemäße Zubereitung sowie die erfindungsgemäße Verwendung.

Sofern nicht anders angegeben wurden alle Versuche unter Normalbedingungen durchgeführt. Der Begriff "Normalbedingungen" bedeutet 20°C, 1013 hPa und eine relative Luftfeuchtigkeit von 50%.

Wird der Begriff Haut verwendet, so bezieht dieser sich bevorzugt auf die menschliche Haut.

Die erfindungsgemäße O/W-Emulsion ist acrylatfrei. Acrylatfrei bedeutet im Sinne der vorliegenden Erfindung, dass der Gesamtanteil an Acrylat-basierten Polymeren weniger als 0,05 Gew.-% und insbesondere bevorzugt 0 Gew-% beträgt, wobei sich die Angaben auf das Gesamtgewicht der Emulsion beziehen. Erfindungsgemäß werden unter Acrylat-basierten Polymeren alle Polymere verstanden, welche aus einer Homo- oder Copolymerisation mit Acryl- und/oder Methacrylsäure gewonnen werden.

Im Sinne der vorliegenden Offenbarung bedeutet silikonfrei, dass keine Moleküle aufweisend Dialkylsiloxan-Einheiten enthalten sind.

Erfindungsgemäß umfasst die kosmetische O/W-Emulsion eine Substanz gewählt aus der Gruppe der Alkalimetallalkylsulfate, wobei die Alkylgruppe jeweils 15 bis 19 Kohlenstoffatome aufweist.

Unter den Alkalimetallalkylsulfaten werden bevorzugt Natriumcetylsulfat, Natriumstearylsulfat und deren Mischung bekannt unter dem Namen Natriumcetearylsulfat eingesetzt.

Ein bevorzugter Gegenstand der Erfindung ist somit eine acrylatfreie und silikonfreie kosmetische O/W-Emulsion enthaltend,
a) mindestens eine Substanz gewählt aus der Gruppe Natriumcetylsulfat, Natriumstearylsulfat und Natriumcetearylsulfat,
b) Hydroxypropylstärkephosphat, und
c) Glyceryl Stearate SE.

Es ist erfindungsgemäß weiterhin vorteilhaft, wenn die jeweils vorstehend unter a) genannten Substanzen in einem Gesamtanteil von 0,05 Gew.-% bis 2,0 Gew.-%, mehr bevorzugt von 0,08 Gew.-% bis 1,5 Gew.-% und am meisten bevorzugt von 0,1 Gew.-% bis 0,3 Gew.-% enthalten sind, bezogen auf das Gesamtgewicht der Emulsion.

Weiterhin ist es vorteilhaft im Sinne der vorliegenden Erfindung, wenn der Anteil von Hydroxypropylstärkephosphat von 0,1 Gew.-% bis 5 Gew.-%, bevorzugt von 0,15 Gew.-% bis 4,0 Gew.-% und insbesondere bevorzugt von 0,2 Gew.-% bis 3 Gew.-%, bezogen auf das Gesamtgewicht der Emulsion, beträgt.

Generell ist Hydroxypropylstärkephosphat ein Veresterungsprodukt basierend auf Stärke. Erfindungsgemäß ist es möglich Hydroxypropylstärkephosphat basierend auf verschiedenen Stärken einzusetzen. Dem Fachmann sind unter anderem Weizen- oder Kartoffelstärke bekannt.

Überraschend hat sich allerdings für den Fachmann herausgestellt, dass, wenn die eingesetzte Hydroxypropylstärkephosphat ein Veresterungsprodukt basierend auf Maisstärke ist, die erfindungsgemäße O/W-Emulsion deutlich weniger klebrig auf der Haut ist, als wenn andere Stärkequellen verwendet werden. Vorteilhafte Ausführungsformen der vorliegenden Erfindung sind somit dadurch gekennzeichnet, dass das eingesetzte Hydroxypropylstärkephosphat ein Veresterungsprodukt basierend auf Maisstärke ist. Innerhalb dieser Ausführungsformen ist es weiter bevorzugt, wenn der Anteil des Hydroxypropylstärkephosphat, welches ein Veresterungsprodukt basierend auf Maisstärke ist, von 0,1 Gew.-% bis 5 Gew.-%, bevorzugt von 0,15 Gew.-% bis 4,0 Gew.-% und insbesondere bevorzugt von 0,2 Gew.-% bis 3 Gew.-%, bezogen auf das Gesamtgewicht der Emulsion, beträgt.

Ein erfindungsgemäßes Hydroxypropylstärkephosphat basierend auf Maisstärke ist unter dem Handelsnamen C*HiForm A 12747 von Cargill oder Structure^{®} XL von Akzo Nobel Specialty Chemicals zu beziehen.

Ferner ist es erfindungsgemäß vorteilhaft, wenn der Gesamtanteil von Glycerin Stearate SE von 0,1 Gew.-% bis 6,0 Gew.-%, bevorzugt 0,5 Gew.-% bis 5,0 Gew.-% und insbesondere bevorzugt von 1,0 Gew.-% bis 3,0 Gew.-%, beträgt, wobei sich die Angaben auf das Gesamtgewicht der Emulsion beziehen.

Weiterhin wurde überraschend gefunden, dass sich insbesondere Emulsionen mit der erfindungsgemäßen Kombination stabilisieren lassen, welche dadurch gekennzeichnet sind, dass der Anteil der Ölphase der Emulsion von mehr als 2 Gew.-% bis 30 Gew.-%, bevorzugt von mehr als 3 Gew.-% bis 20 Gew.-% und insbesondere bevorzugt von mehr als 4 Gew.-% bis 15,5 Gew.-%, bezogen auf das Gesamtgewicht der Emulsion, beträgt, wobei Tenside und Emulgatoren definitionsgemäß nicht zur Ölphase zählen, es sei denn sie sind in der vorliegenden Offenbarung explizit der Ölphase zugeordnet. Somit lassen sich überraschender Weise Emulsionen mit einem großen Ölphasen-Anteil stabilisieren.

Erfindungsgemäß zählen Emulgatoren und Tenside nicht zur Ölphase. Das heißt, dass unter anderem Glyceryl Stearate SE und Natriumalkylsulfate nicht zur Ölphase zählen.

Unter Emulgatoren werden alle Substanzen verstanden, welche im International Cosmetic Ingredient Dictionary and Handbook, Thirteenth Edition 2010, (ISBN 1-882621-47-6) unter der Bezeichnung "emulsifying agent" geführt werden. Unter Tensiden werden alle Substanzen verstanden, welche im International Cosmetic Ingredient Dictionary and Handbook, Thirteenth Edition 2010, (ISBN 1-882621-47-6) unter der Bezeichnung " surfactant " geführt werden.

Zusätzlich ist es vorteilhaft, wenn neben dem Emulgator Glyceryl Stearate SE zusätzlich auch der Emulgator Glyceryl Stearate enthalten ist, wobei der der Anteil von Glyceryl Stearate bevorzugt von 0,3 bis 7 Gew.-%, weiterhin bevorzugt von 0,35 bis 5 Gew.-% und insbesondere bevorzug von 0,5 bis 1,5 Gew.-%, bezogen auf das Gesamtgewicht der Emulsion, beträgt.

Es hat sich weiterhin überraschend gezeigt, dass die Temperaturstabilität der erfindungsgemäßen O/W-Emulsion weiter gesteigert werden konnte, indem der Emulsion ein oder mehrere Fettalkohole mit 14 bis 22 Kohlenstoffatomen zugesetzt werden. Der Gesamtanteil dieser Fettalkohole mit 14 bis 22 Kohlenstoffatomen beträgt vorteilhaft von 0,1 Gew.-% bis 7,0 Gew.-%, bevorzugt 0,5 Gew.-% bis 6,0 Gew.-% und insbesondere bevorzugt von 1 Gew.-% bis 5,0 Gew.-%,bezogen auf das Gesamtgewicht der Emulsion. Fettalkohole mit 14 bis 22 Kohlenstoffatomen zählen zur Ölphase.

Insbesondere bevorzugt zu wählende Fettalkohole sind Cetylalkohol und/oder Stearylalkohol, wobei diese bevorzugt in den Anteilen von 0,1 Gew.-% bis 7,0 Gew.-%, bevorzugt 2 Gew.-% bis 6,0 Gew.-% und insbesondere bevorzugt von 3 Gew.-% bis 5,0 Gew.-%, bezogen auf das Gesamtgewicht der Emulsion, eingesetzt werden. Die Mischung aus Cetylalkohol und Stearylalkohol ist unter dem INCI Namen Cetearylalkohol bekannt.

Vorteilhafte Ausführungsformen der vorliegenden Erfindung sind weiterhin dadurch gekennzeichnete, dass die O/W-Emulsion weitere Polysaccharid haltige Polymere enthält.

Vorteilhaft enthält die O/W-Emulsion dieser Ausführungsformen Tapiokastärke, wobei der Anteil von Tapiokastärke bevorzugt im Bereich von 0,5 Gew.-% bis 3,0 Gew.-% liegt, wobei sich die Gewichtsangaben auf das Gesamtgewicht der O/W-Emulsion beziehen.

Weiterhin enthält die O/W-Emulsion dieser Ausführungsformen vorteilhaft ein oder mehrere weitere Stärkephosphate, wobei der Gesamtanteil dieser zusätzlichen Stärkephosphate bevorzugt im Bereich von 0,5 Gew.-% bis 3,0 Gew.-% liegt, wobei sich die Gewichtsangaben auf das Gesamtgewicht der O/W-Emulsion beziehen. Unter dem Begriff "weiteren Stärkephosphaten" sind Stärkephosphate zu verstehen, welche von Hydroxypropylstärke verschieden sind.

Weiterhin enthält die O/W-Emulsion dieser Ausführungsformen vorteilhaft Xanthan und/oder ein modifiziertes Xanthan, wobei der Gesamtanteil dieser Polymere im Bereich von 0,05 Gew.-% bis 0,5 Gew.-% liegt, wobei sich die Gewichtsangaben auf das Gesamtgewicht der O/W-Emulsion beziehen.

Ferner enthält die O/W-Emulsion dieser Ausführungsformen vorteilhaft Carrageenan, wobei der Gesamtanteil von Carrageenan im Bereich von 0,05 Gew.-% bis 0,5 Gew.-% liegt, wobei sich die Gewichtsangaben auf das Gesamtgewicht der O/W-Emulsion beziehen.

Andere vorteilhafte Ausführungsformen der vorliegenden Erfindung sind ferner dadurch gekennzeichnet, dass diese neben Hydoxypropylstärkephosphat keine weiteren Stärken und Stärkederivate enthält.

Andere vorteilhafte Ausführungsformen der vorliegenden Erfindung sind ferner dadurch gekennzeichnet, dass diese keine Cellulosen oder Cellulosederivate enthält.

Andere vorteilhafte Ausführungsformen der vorliegenden Erfindung sind ferner dadurch gekennzeichnet, dass diese keine weiteren Polysaccharid-haltige Polymere enthält.

Die erfindungsgemäße O/W-Emulsion enthält weiterhin vorteilhaft ein oder mehrere Lipidkomponenten, wie Wachse und Öle auf Basis von Kohlenwasserstoffen, gesättigte, ungesättige oder gehärtete Triglyceride, Dialkylether mit 12 bis 24 Kohlenstoffatomen und/oder die Ester aus einwertigen Alkoholen und Monocarbonsäuren, welche mindestens 10 Kohlenstoffatome aufweisen.

Wie bereits ausgeführt, ist es vorteilhaft, wenn die O/W-Emulsion der vorliegenden Erfindung kein Mineralöl enthält.

Ferner ist es vorteilhaft, wenn keine verzweigten und/oder unverzweigten Kohlenwasserstoffe enthalten sind. Beispiele derartiger Kohlenwasserstoffe sind Paraffinum Liquidum, Isododecan, Isohexadecan, Isoeicosane, Squalan und Cera Microcristallina.

Die erfindungsgemäße O/W-Emulsion enthält vorteilhaft ein oder mehrere Triglycerinester, gewählt aus der Gruppe der synthetischen, halbsynthetischen und natürlichen Triglycerinester.

Vorteilhaft enthaltene natürliche Öle sind gewählt aus der Gruppe Persea Gratissima Oil, Orbignya Oleifera Seed Oil, Argania Spinosa Kernel Oil, Prunus Armeniaca Kernel Oil, Simmondsia Chinensis Seed Oil, Cocos Nucifera Oil, Silybum Marianum Seed Oil, Oenothera Biennis Oil, Olea Europaea Fruit Oil, Helianthus Annuus Seed Oil, Vitis Vinifera Seed Oil, Cannabis Sativa Seed Oil, Vegetable Oil, Gossypium Herbaceum Seed Oil, Arctium Lappa Seed Oil, Macadamia Ternifolia Seed Oil, Macadamia Integrifolia Seed Oil, Zea Mays Germ Oil, Prunus Amygdalus Dulcis Oil, Ricinus Communis Seed Oil, vegetable Oil und Glycine Soja Oil.

Enthält die O/W-Emulsion ein oder mehrere der vorstehend genannten natürlichen Öle, so beträgt der Anteil dieser natürlichen Öle bevorzugt von 0,1 Gew.-% bis 5,0 Gew.-%, bezogen auf das Gesamtgewicht der O/W-Emulsion.

Es ist ebenfalls vorteilhaft, wenn ein Öl gewählt aus der Gruppe Coco-Caprylate, Dicaprylyl Ether, Cocoglycerides, Coco-Caprylate/Caprate, Decyl Oleate, Caprylic/ CapricTriglyceride, Ethylhexyl Cocoate, Octyldodecanol, Squalane, Triisostearin, Shea Butter Ethyl Esters, Ethylhexyl Cocoate, Decyl Cocoate, Isoamyl Cocoate, Caprylyl Caprylate/ Caprate, Triheptanoin, Hexyldecyl Sterate und Isoamyl Laurate enthalten ist.

Zu den weiteren bevorzugten Triglycerinestern zählen unter anderen gehärtete Triglyceridfette, wie hydriertes Palmöl, hydriertes Kokosöl oder hydriertes Rizinusöl. Insbesondere ist der Einsatz von hydriertem Kokosöl (Hydrogenated Coco-Glycerides) bevorzugt, wobei der Anteil von hydriertem Kokosöl bevorzugt von 0,5 Gew.-% bis 3 Gew.-%, bezogen auf das Gesamtgewicht der Emulsion, beträgt.

Weiterhin ist es insbesondere vorteilhaft, wenn die Emulsion Capryl-Caprinsäuretriglycerid enthält, wobei der Anteil von Capryl-Caprinsäuretriglycerid bevorzugt von 0,5 Gew.-% bis 5,0 Gew.-%, bezogen auf das Gesamtgewicht der O/W-Emulsion beträgt.

Es ist ebenfalls insbesondere von Vorteil, wenn die Emulsion Cocoglycerides enthält, wobei der Anteil von Cocoglycerides bevorzugt von 0,5 bis 9 Gew.-% und insbesondere bevorzugt von 1 bis 8 Gew.-% bezogen auf das Gesamtgewicht der Emulsion beträgt.

Ferner ist es vorteilhaft im Sinne der vorliegenden Erfindung, wenn die O/W-Emulsion der vorliegenden Erfindung ein oder mehrere Dialkylether mit 12 bis 24 Kohlenstoffatomen enthält, wobei bevorzugt Dicaprylylether enthalten ist. Enthält die O/W-Emulsion Dicaprylylether, so beträgt der Anteil von Dicaprylylether bevorzugt von 0,5 Gew.-% bis 3 Gew.-%, bezogen auf das Gesamtgewicht der O/W-Emulsion.

Weiterhin ist es insbesondere vorteilhaft im Sinne der vorliegenden Erfindung, wenn die O/W-Emulsion der vorliegenden Erfindung ein oder mehrere Ester aus einwertigen Alkoholen und Monocarbonsäuren enthält, wobei die Ester mindestens 10, bevorzugt mindestens 15 Kohlenstoffatome aufweisen. Vorteilhaft zu wählende Ester aus einwertigen Alkoholen und Monocarbonsäuren sind 2-Ethylhexylisostearat, Isotridecylisononanoat, 2-Ethylhexylcocoat, C₁₂₋₁₅-Alkylbenzoat, Isopropylmyristat und Isopropylpalmitat.

Es ist weiterhin vorteilhaft im Sinne der vorliegenden Erfindung, wenn die O/W-Emulsion Octyldodecanol enthält.

Ferner ist es insbesondere vorteilhaft, wenn die Emulsion dadurch gekennzeichnet ist, dass sie Coco-Caprylate/Caprate enthält, wobei der Anteil von Coco-Caprylate/Caprate bevorzugt von 0,5 bis 7 Gew.-% und insbesondere bevorzugt von 2 bis 6 Gew.-% bezogen auf das Gesamtgewicht der Emulsion beträgt.

Ein weiterer insbesondere vorteilhafter Bestandteil der der Emulsion ist weiterhin Butyrospermum Parkii Butter, welche bevorzugt in ein Anteil von 0,3 bis 2 Gew.-% und insbesondere bevorzugt von 0,5 bis 1,5 Gew.-% bezogen auf das Gesamtgewicht der Emulsion enthalten ist.

Es ist darüber hinaus erfindungsgemäß von Vorteil, wenn die O/W-Emulsion Propylenglycol, Butylenglycol, 2-Methylpropan-1,3-diol, 1,2-Pentandiol, 1,2-Hexandiol, 1,2-Octandiol Glycerylcaprylate und/oder 1,2-Decandiol enthält.

Weiterhin ist es ebenfalls vorteilhaft im Sinne der vorliegenden Erfindung, wenn die O/W-Emulsion Phenoxyethanol, Dehydracetsäure, Benzyl Alkohol und/oder Ethylhexylglycerin enthält.

Enthält die O/W-Emulsion Benzylalkohol so ist es bevorzugt, wenn der Anteil an Benzylalkohol von 0,1 Gew.-% bis 0,5 Gew.-% beträgt, bezogen auf das Gesamtgewicht der O/W-Emulsion.

Enthält die O/W-Emulsion Phenoxyethanol, so ist es bevorzugt, wenn der Gesamtanteil an Phenoxyethanol von 0,1 Gew.-% bis 1,2 Gew.-% beträgt, bezogen auf das Gesamtgewicht der O/W-Emulsion.

Enthält die O/W-Emulsion Ethylhexylglycerin, so ist es bevorzugt, wenn der Anteil an Ethylhexylglycerin von 0,1 Gew.-% bis 1,0 Gew.-% beträgt, bezogen auf das Gesamtgewicht der O/W-Emulsion.

Darüber hinaus ist es vorteilhaft, wenn Ausführungsformer der Erfindung dadurch gekennzeichnet sind, dass diese Ethanol enthalten. Ist Ethanol in der O/W-Emulsion enthalten, so beträgt der Anteil von Ethanol bevorzugt von 0,5 Gew.-% bis 10 Gew.-%, bezogen auf das Gesamtgewicht der O/W-Emulsion.

Ferner ist die erfindungsgemäße O/W-Emulsion bevorzugt dadurch gekennzeichnet, dass diese Glycerin in einem Anteil von 0,5 Gew.-% bis 15% Gew.-% , bezogen auf das Gesamtgewicht der O/W-Emulsion, enthält.

Weiterhin, ist es bevorzugt, wenn die O/W-Emulsion weitere Tenside und/oder Emulgatoren nur in einem Maximalanteil von 2 Gew.-%, bevorzugt in einem Maximalanteil von 1,5 Gew.-% enthält, wobei sich die Angaben auf das Gesamtgewicht der Emulsion beziehen.

Nicht zuletzt sind erfindungsgemäß vorteilhafte Ausführungsformen dadurch gekennzeichnet, dass die O/W-Emulsion einen oder mehrere Wirkstoffe gewählt aus der Gruppe der Verbindungen Glycyrrhetinsäure, Harnstoff, Arctiin, Folsäure, Coenzym Q10 (Ubiquinon), alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, Vitamin C, Vitamin C phosphate, Vitamin C palmitate, Niacinamide, Vitamin A palmitate, Panthenol, Licochalcon A, Rucinol, N-[(2,4-Dihydroxyphenyl)thiazol-2-yl]isobutyramide, Honociol und Magnolol (auch als Bestandteil von Magnolia-Extrakten), Hyaluronsäure und/oder Silymarin (Mariendistelextrakt) enthält.

Ferner sind erfindungsgemäß vorteilhafte O/W-Emulsionen dadurch gekennzeichnet, dass diese Wasser in einem Anteil von 60 Gew.-% bis 95 Gew.-% und bevorzugt von 70 Gew.-% bis 90 Gew.-%, bezogen auf das Gesamtgewicht der Emulsion enthalten.

Weiterhin weisen erfindungsgemäß vorteilhafte O/W-Emulsionen 24 h nach Herstellung eine Viskosität von 3000 mPa·s bis 6000 mPa·s auf. Wird in dieser Offenbarung von Viskosität gesprochen, so beziehen sich alle Werte auf eine Messung bei 25°C im 150 ml Rollrandglas mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messystems wird die Viskosität berechnet. Als Messkörper wird der Messkörper Nr.1 (Artikelnr. 200 0191), Drehzahl Bereich 62,5 min⁻¹, verwendet. Alle Messungen zur Viskosität erfolgen immer 24h nach Herstellung der O/W-Emulsion.

### Vergleichsversuche und Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Die nachfolgende Tabelle zeigt mit Vgl. 1 und Vgl.2 zwei nicht erfindungsgemäße Emulsionen, während Bsp.1 eine erfindungsgemäße Emulsion darstellt. Die Eigenschaften von Vgl.1 und Vgl.2 wurden zu Bsp.1 verglichen. Ein Expertenpanel hat beim Auftragen der Emulsionen auf die Haut festgestellt, dass Vgl. 1 und Vgl.2 signifikante Mengen an weißen Rückständen beim Auftragen auf dem Unterarm hinterlassen. Hingegen zeigt Bsp.1 fast keine weißen Rückstände.

| **Inhaltstoff** | **Vgl.1** | **Vgl.2** | **Bsp.1** |
|---|---|---|---|
| Sodium Cetearyl Sulfate | 0,15 | 0,15 | 0,15 |
| Glyceryl Stearate SE | 1,5 | 1,5 | 1,5 |
| Glyceryl Stearate | 1 | 1 | 1 |
| Cetearyl Alcohol | 4 | 4 | 4 |
| Butyrospermum Parkii Butter | 1 | 1 | 1 |
| Cocoglycerides | 6 | 6 | 6 |
| Coco-Caprylate | 4 | 4 | 4 |
| Carbomer | 0,1 | | |
| Hydroxypropyl Starch Phosphate | | | 1 |
| Xanthan Gum | | 0,5 | |
| Glycerin | 7 | 7 | 7 |
| Phenoxyethanol | 0,9 | 0,9 | 0,9 |
| Ethylhexylglycerin | 0,2 | 0,2 | 0,2 |
| Natronlauge 45% -ig | 0,06 | | |
| Aqua | ad 100 | ad 100 | ad 100 |
| Weiße Rückstände nach auftragen auf dem Unterarm | stark | sehr stark | kaum |
| Viskosität 24 h nach Herstellung, 25°C | 4650 mPas | 3250 mPas | 4550 mPas |
| Stabilität, Lagerung bei 40°C für 1 Monat | i.O. | i.O. | i.O. |
| Stabilität, Lagerung bei 50°C 1 Monat | i.O. | starke Wasserabscheidung | i.O. |

Die Abkürzung i.O. steht vorstehend für in Ordnung, es wurde keine Phasentrennung festgestellt.

Die weiteren Beispiele sollen die Erfindung weiter verdeutlichen ohne sie einzuschränken.

| **Inhaltstoffe** | **Bsp.2** | **Bsp.3** | **Bsp.4** | **Bsp.5** | **Bsp.6** | **Bsp.7** |
|---|---|---|---|---|---|---|
| Sodium Cetearyl Sulfate | 0,15 | 0,25 | 0,15 | 0,15 | 0,2 | 0,05 |
| Glyceryl Stearate SE | 1,5 | 3 | 3 | 1,5 | 1,5 | 2,5 |
| Glyceryl Stearate | 1 | 1 | | 1 | 1 | 1 |
| Cetearyl Alcohol | 4 | 3 | 3,5 | | 4 | 4 |
| Stearylalkohol | | | | 3 | | |
| Butyrospermum Parkii Butter | 1 | 1 | | 1 | | 1 |
| Coco-Caprylate/Caprate | | 3 | 2 | | | |
| Cocoglycerides | 3 | | 6 | 1 | | 6 |
| Coco-Caprylate | | 4 | | 4 | 4 | 4 |
| Helianthus Annuus Seed Oil | 2 | | | | | |
| Dicaprylyl Ether | 3 | 2 | 2 | | | |
| Ethylhexyl Cocoate | | | | 3 | 2 | |
| Hydroxypropyl Starch Phosphate | 1 | 0,5 | 1 | 0,7 | 1,5 | 2 |
| Glycerin | 7 | 8 | 5 | 6 | 5 | 7 |
| Phenoxyethanol | 0,9 | 0,9 | | 0,9 | | 0,5 |
| Ethylhexylglycerin | 0,2 | 0,2 | | | 0,2 | |
| Glyceryl Caprylate | | | 0,3 | | | 0,3 |
| Glyceryl Caprate | | | | 0,5 | | |
| Alcohol | | | 3,0 | | | |
| Perfume | 0,2 | | | 0,4 | | 0,3 |
| Aqua | ad 100 | ad 100 | ad 100 | ad 100 | ad 100 | ad 100 |

| **Inhaltstoffe** | **Bsp.8** | **Bsp.9** | **Bsp.10** | **Bsp.11** | **Bsp.12** | **Bsp.13** |
|---|---|---|---|---|---|---|
| Sodium Cetearyl Sulfate | 0,15 | 0,1 | 0,15 | 0,2 | 0,1 | 0,15 |
| Glyceryl Stearate SE | 2 | 2,5 | 3,0 | 1,5 | 2,5 | 2,5 |
| Glyceryl Stearate | | | | 1 | | |
| Cetearyl Alcohol | 4 | 4 | 3,5 | 2,5 | 5 | 3 |
| Cetyl Palmitate | | | 1,0 | | | |
| Dicaprylyl Ether | | 3 | | | | |
| Caprylic/Capric Triglyceride | | | | 4 | 3 | |
| Coco-Caprylate/Caprate | 4 | | 3 | | 2 | |
| Vegetable Oil | | | 4 | | | 3 |
| Theobroma Cacao Seed Butter | | | 1 | | | |
| Octyldodecanol | 3 | 3,0 | | | | |
| Undecane / Tridecane | | | | | | 3 |
| Hydroxypropyl Starch Phosphate | 0,4 | 0,5 | 0,5 | 0,8 | 1 | 0,7 |
| Glycerin | 5 | 6 | 7 | 7 | 5 | 8 |
| Phenoxyethanol | 0,5 | | 1 | | 0,8 | |
| Alcohol Denat. | | 2 | 2 | 2 | | 1 |
| Citric Acid | 0,2 | | | 0,3 | | |
| Caprylyl Glycol | | 1,0 | | | | |
| Ethylhexylglycerin | | | 0,5 | | | |
| Benzylalkohol | | | | | 0,3 | |
| Sodium Phytate | | | 0,2 | | | |
| Perfume | | | 0,4 | | | |
| Aqua | ad 100 | ad 100 | ad 100 | ad 100 | ad 100 | ad 100 |

| **Inhaltstoffe** | **Bsp.14** | **Bsp. 15** | **Bsp.16** |
|---|---|---|---|
| Sodium Cetearyl Sulfate | 0,2 | 0,15 | 0,15 |
| Glyceryl Stearate SE | 1,5 | 1,5 | 1 |
| Glyceryl Stearate | | 3 | 5 |
| Cetearyl Alcohol | | 4,5 | |
| Cetyl Alcohol | 2 | | |
| Stearyl Alcohol | 2 | | 4 |
| Caprylic/Capric Triglyceride | | | 2 |
| Octyldodecanol | 3 | | |
| Tiisostearin | | 1 | |
| Prunus Amygdalus Dulcis Oil | | 2 | |
| Vegetable oil | 0,9 | 0,4 | 3 |
| Hydrogenated Coco-Glycerides | 1 | 0,5 | 1 |
| Butyrospermum Parkii Butter | 0,5 | | 1 |
| Hxdroxypropyl Starch Phosphate | 0,75 | 1 | 2 |
| Tapioca Starch | | | 2 |
| Xanthan Gum | | | 0,1 |
| Ethylhexylglycerin | 0,5 | 0,3 | 0,1 |
| Glycerin | 8 | 5 | 15 |
| Alcohol | 3 | 4 | 5 |
| Parfum | 0,2 | 0,3 | 0,4 |
| Aqua | ad 100 | ad 100 | ad 100 |

## Patentansprüche

1. Acrylat- und silikonfreie kosmetische O/W-Emulsion enthaltend
a) mindestens eine Substanz gewählt aus der Gruppe der Alkalimetallalkylsulfate, wobei die Alkylgruppe jeweils 15 bis 19 Kohlenstoffatome aufweist,
b) Hydroxypropylstärkephosphat, und
c) Glyceryl Stearate SE,
**dadurch gekennzeichnet, dass** die folgenden Rezepturen ausgeschlossen sind:
| | **B1** | **D1** | **E1** | **B2** | **D2** | **E2** | **F2** |
|---|---|---|---|---|---|---|---|
| **INCI** | **m [%]** | **m [%]** | **m [%]** | **m [%]** | **m [%]** | **m [%]** | **m [%]** |
| Hydroxypropyl Starch Phosphate | 0,5 | 1,5 | 0,75 | 0,5 | 0,7 | 1 | 2,5 |
| Coco-Caprylate/Caprate | | | 3 | | 3 | | |
| Coco-Caprylate | | | | 4 | | | |
| Decyl Cocoate | | | | | | | 3 |
| Simmondsia Chinensis Seed Oil | | | 3 | | | | |
| Vegetable Oil | | 4 | | | | | |
| Shea Butter Ethyl Ester | | | | | | | 3 |
| Caprylic/Capric Triglyceride | | | | | | 3 | |
| Dicaprylyl Ether | 4 | 2 | 1,5 | | 1,5 | | |
| Prunus Amygdalus Dulcis Oil | | | | | | 2 | |
| Brassica Campestris Seed Oil | 4 | | | | | | |
| Cocos Nucifera Oil | | | | | | | 0,5 |
| Cocoglyceride | | | | 6 | 3 | | |
| Theobroma Cacao Seed Butter | | | 1 | | | | 1 |
| Sodium Cetearyl Sulfate | 0,15 | 0,1 | 0,15 | 0,15 | 0,15 | 0,15 | 0,2 |
| Sodium Stearoyl Glutamate | | | | | | | |
| Glyceryl Stearate SE | 1,5 | 1,7 | 2,5 | 1,5 | 2 | 2 | 2,5 |
| Glyceryl Stearate | 1 | | 0,5 | 1 | 1 | | 0,5 |
| Cetearyl Alcohol | 4 | 1,2 | | 4 | 4 | 2 | |
| Stearylalkohol | | | 3 | | | | |
| Butyrospermum Parkii Butter | 1 | 1 | | 1 | 1 | | |
| Cetyl Palmitate | | | 0,5 | | | 1 | |
| Glycerin | 7 | 7 | 7 | 7 | 7 | 7 | 5 |
| Phenoxyethanol | 0,9 | | 0,9 | 0,9 | 0,5 | | |
| Ethylhexylglycerin | 0,2 | 0,2 | 0,2 | 0,2 | 0,5 | 0,3 | |
| Benzylalcohol | | 0,2 | | | | 0,2 | |
| Polyglyceryl-2 Caprate | | | | | | | 0,5 |
| Alcohol | | 4 | 4 | | 3 | | 4 |
| Perfume | | 0,2 | | | 0,4 | | 0,3 |
| Aqua | Ad 100 | ad 100 | ad 100 | Ad 100 | Ad 100 | Ad 100 | Ad 100 |

2. Verwendung eine Mischung aus
a) mindestens eine Substanz gewählt aus der Gruppe der Alkalimetallalkylsulfate, wobei die Alkylgruppe jeweils 15 bis 19 Kohlenstoffatome aufweist,
b) Hydroxypropylstärkephosphat, und
c) Glyceryl Stearate SE
in einer acrylat- und silikonfreien kosmetische O/W-Emulsion zur Reduzierung von weißen Rückständen beim Auftragen der Emulsion auf die Haut,
**dadurch gekennzeichnet, dass** die Folgenden Rezepturen ausgeschlossen sind:
| | **B1** | **D1** | **E1** | **B2** | **D2** | **E2** | **F2** |
|---|---|---|---|---|---|---|---|
| **INCI** | **m [%]** | **m [%]** | **m [%]** | **m [%]** | **m [%]** | **m [%]** | **m [%]** |
| Hydroxypropyl Starch Phosphate | 0,5 | 1,5 | 0,75 | 0,5 | 0,7 | 1 | 2,5 |
| Coco-Caprylate/Caprate | | | 3 | | 3 | | |
| Coco-Caprylate | | | | 4 | | | |
| Decyl Cocoate | | | | | | | 3 |
| Simmondsia Chinensis Seed Oil | | | 3 | | | | |
| Vegetable Oil | | 4 | | | | | |
| Shea Butter Ethyl Ester | | | | | | | 3 |
| Caprylic/Capric Triglyceride | | | | | | 3 | |
| Dicaprylyl Ether | 4 | 2 | 1,5 | | 1,5 | | |
| Prunus Amygdalus Dulcis Oil | | | | | | 2 | |
| Brassica Campestris Seed Oil | 4 | | | | | | |
| Cocos Nucifera Oil | | | | | | | 0,5 |
| Cocoglyceride | | | | 6 | 3 | | |
| Theobroma Cacao Seed Butter | | | 1 | | | | 1 |
| Sodium Cetearyl Sulfate | 0,15 | 0,1 | 0,15 | 0,15 | 0,15 | 0,15 | 0,2 |
| Sodium Stearoyl Glutamate | | | | | | | |
| Glyceryl Stearate SE | 1,5 | 1,7 | 2,5 | 1,5 | 2 | 2 | 2,5 |
| Glyceryl Stearate | 1 | | 0,5 | 1 | 1 | | 0,5 |
| Cetearyl Alcohol | 4 | 1,2 | | 4 | 4 | 2 | |
| Stearylalkohol | | | 3 | | | | |
| Butyrospermum Parkii Butter | 1 | 1 | | 1 | 1 | | |
| Cetyl Palmitate | | | 0,5 | | | 1 | |
| Glycerin | 7 | 7 | 7 | 7 | 7 | 7 | 5 |
| Phenoxyethanol | 0,9 | | 0,9 | 0,9 | 0,5 | | |
| Ethylhexylglycerin | 0,2 | 0,2 | 0,2 | 0,2 | 0,5 | 0,3 | |
| Benzylalcohol | | 0,2 | | | | 0,2 | |
| Polyglyceryl-2 Caprate | | | | | | | 0,5 |
| Alcohol | | 4 | 4 | | 3 | | 4 |
| Perfume | | 0,2 | | | 0,4 | | 0,3 |
| Aqua | Ad 100 | ad 100 | ad 100 | Ad 100 | Ad 100 | Ad 100 | Ad 100 |

3. Emulsion nach Anspruch 1 oder Verwendung nach Anspruch 2 **dadurch gekennzeichnet, dass** die Alkalimetallalkylsulfate gewählt sind aus der Gruppe Natriumcetylsulfat, Natriumstearylsulfat und Natriumcetearylsulfat.

4. Emulsion oder Verwendung nach einem der vorhergehenden Ansprüche **dadurch gekennzeichnet, dass** der Gesamtanteil der Alkalimetallalkylsulfate von 0,05 Gew.-% bis 2,0 Gew.-%, mehr bevorzugt von 0,08 Gew.-% bis 1,5 Gew.-% und am meisten bevorzugt von 0,1 Gew.-% bis 0,3 Gew.-% enthalten sind, bezogen auf das Gesamtgewicht der Emulsion.

5. Emulsion oder Verwendung nach einem der vorhergehenden Ansprüche **dadurch gekennzeichnet, dass** der Anteil von Hydroxypropylstärkephosphat von 0,1 Gew.-% bis 5 Gew.-%, bevorzugt von 0,15 Gew.-% bis 4,0 Gew.-% und insbesondere bevorzugt von 0,2 Gew.-% bis 3 Gew.-%, bezogen auf das Gesamtgewicht der Emulsion, beträgt.

6. Emulsion oder Verwendung nach einem der vorhergehenden Ansprüche **dadurch gekennzeichnet, dass** der Gesamtanteil von Glycerin Stearate SE in der Emulsion von 0,1 Gew.-% bis 6,0 Gew.-%, bevorzugt 0,5 Gew.-% bis 5,0 Gew.-% und insbesondere bevorzugt von 1,0 Gew.-% bis 3,0 Gew.-%, beträgt, wobei sich die Angaben auf das Gesamtgewicht der Emulsion beziehen.

7. Emulsion oder Verwendung nach einem der vorhergehenden Ansprüche **dadurch gekennzeichnet, dass** der Anteil der Ölphase der Emulsion von mehr als 2 Gew.-% bis 30 Gew.-%, bevorzugt von mehr als 3 Gew.-% bis 20 Gew.-% und insbesondere bevorzugt von mehr als 4 Gew.-% bis 15,5 Gew.-%, bezogen auf das Gesamtgewicht der Emulsion, beträgt.

8. Emulsion oder Verwendung nach einem der vorhergehenden Ansprüche **dadurch gekennzeichnet, dass** die Emulsion Glyceryl Stearate enthält, wobei der der Anteil von Glyceryl Stearate bevorzugt von 0,3 bis 7 Gew.-%, weiterhin bevorzugt von 0,35 bis 5 Gew.-% und insbesondere bevorzug von 0,5 bis 1,5 Gew.-%, bezogen auf das Gesamtgewicht der Emulsion, beträgt.

9. Emulsion oder Verwendung nach einem der vorhergehenden Ansprüche **dadurch gekennzeichnet, dass** die Emulsion ein oder mehrere Fettalkohole mit 14 bis 22 Kohlenstoffatomen enthält, wobei der Gesamtanteil dieser Fettalkohole mit 14 bis 22 Kohlenstoffatomen vorteilhaft von 0,1 Gew.-% bis 7,0 Gew.-%, bevorzugt 0,5 Gew.-% bis 6,0 Gew.-% und insbesondere bevorzugt von 1 Gew.-% bis 5,0 Gew.-%, bezogen auf das Gesamtgewicht der Emulsion, beträgt.

10. Emulsion oder Verwendung nach einem der vorhergehenden Ansprüche **dadurch gekennzeichnet, dass** die O/W-Emulsion der vorliegenden Erfindung kein Mineralöl enthält.

11. Emulsion oder Verwendung nach einem der vorhergehenden Ansprüche **dadurch gekennzeichnet, dass** die Emulsion Cocoglycerides enthält, wobei der Anteil von Cocoglycerides bevorzugt von 0,5 bis 9 Gew.-% und insbesondere bevorzugt von 1 bis 8 Gew.-%, bezogen auf das Gesamtgewicht der Emulsion, beträgt.

12. Emulsion oder Verwendung nach einem der vorhergehenden Ansprüche **dadurch gekennzeichnet, dass** die Emulsion Coco-Caprylate/Caprate enthält, wobei der Anteil von Coco-Caprylate/Caprate bevorzugt von 0,5 bis 7 Gew.-% und insbesondere bevorzugt von 2 bis 6 Gew.-% bezogen auf das Gesamtgewicht der Emulsion beträgt.

13. Emulsion oder Verwendung nach einem der vorhergehenden Ansprüche **dadurch gekennzeichnet, dass** die Emulsion Butyrospermum Parkii Butter enthält, wobei Butyrospermum Parkii Butter bevorzugt in einem Anteil von 0,3 bis 2 Gew.-% und insbesondere bevorzugt von 0,5 bis 1,5 Gew.-% bezogen auf das Gesamtgewicht der Emulsion enthalten ist.

14. Emulsion oder Verwendung nach einem der vorhergehenden Ansprüche **dadurch gekennzeichnet, dass** die Emulsion weitere Tenside und/oder Emulgatoren nur in einem Maximalanteil von 2 Gew.-%, bevorzugt in einem Maximalanteil von 1,5 Gew.-% enthält, wobei sich die Angaben auf das Gesamtgewicht der Emulsion beziehen.

15. Emulsion oder Verwendung nach einem der vorhergehenden Ansprüche **dadurch gekennzeichnet, dass** die Emulsionen 24 h nach Herstellung bei 25°C eine Viskosität von 3000 mPa·s bis 6000 mPa·s aufweist, wobei sich diese Angaben auf eine Messung mit einem Rheomat R 123 der Firma ProRheo mit dem Messkörper 1 bei einer Drehzahl von 62,5 min⁻¹ beziehen.

## Claims

1. Acrylate-free and silicone-free cosmetic O/W emulsion comprising
a) at least one substance selected from the group of alkali metal alkyl sulfates, wherein the alkyl group each comprises 15 to 19 carbon atoms,
b) hydroxypropyl starch phosphate, and
c) glyceryl stearate SE,
**characterized in that** the following formulations are excluded:
| | **B1** | **D1** | **E1** | **B2** | **D2** | **E2** | **F2** |
|---|---|---|---|---|---|---|---|
| **INCI** | **m [%]** | **m [%]** | **m [%]** | **m [%]** | **m [%]** | **m [%]** | **m [%]** |
| Hydroxypropyl starch phosphate | 0.5 | 1.5 | 0.75 | 0.5 | 0.7 | 1 | 2.5 |
| Coco-caprylate/caprate | | | 3 | | 3 | | |
| Coco-caprylate | | | | 4 | | | |
| Decyl cocoate | | | | | | | 3 |
| Simmondsia chinensis seed oil | | | 3 | | | | |
| Vegetable oil | | 4 | | | | | |
| Shea butter ethyl esters | | | | | | | 3 |
| Caprylic/capric triglycerides | | | | | | 3 | |
| Dicaprylyl ether | 4 | 2 | 1.5 | | 1.5 | | |
| Prunus amygdalus dulcis oil | | | | | | 2 | |
| Brassica campestris seed oil | 4 | | | | | | |
| Cocos nucifera oil | | | | | | | 0.5 |
| Coco-glycerides | | | | 6 | 3 | | |
| Theobroma cacao seed butter | | | 1 | | | | 1 |
| Sodium cetearyl sulfate | 0.15 | 0.1 | 0.15 | 0.15 | 0.15 | 0.15 | 0.2 |
| Sodium stearoyl glutamate | | | | | | | |
| Glyceryl stearate SE | 1.5 | 1.7 | 2.5 | 1.5 | 2 | 2 | 2.5 |
| Glyceryl stearate | 1 | | 0.5 | 1 | 1 | | 0.5 |
| Cetearyl alcohol | 4 | 1.2 | | 4 | 4 | 2 | |
| Stearyl alcohol | | | 3 | | | | |
| Butyrospermum parkii butter | 1 | 1 | | 1 | 1 | | |
| Cetyl palmitate | | | 0.5 | | | 1 | |
| Glycerol | 7 | 7 | 7 | 7 | 7 | 7 | 5 |
| Phenoxyethanol | 0.9 | | 0.9 | 0.9 | 0.5 | | |
| Ethylhexylglycerin | 0.2 | 0.2 | 0.2 | 0.2 | 0.5 | 0.3 | |
| Benzyl alcohol | | 0.2 | | | | 0.2 | |
| Polyglyceryl-2 caprate | | | | | | | |
| Alcohol | | 4 | 4 | | 3 | | 4 |
| Perfume | | 0.2 | | | 0.4 | | 0.3 |
| Aqua | to 100 | to 100 | to 100 | to 100 | to 100 | to 100 | to 100 |

2. Use of a mixture composed of
a) at least one substance selected from the group of alkali metal alkyl sulfates, wherein the alkyl group each comprises 15 to 19 carbon atoms,
b) hydroxypropyl starch phosphate, and
c) glyceryl stearate SE
in an acrylate-free and silicone-free cosmetic O/W emulsion for reducing white residues when the emulsion is applied to the skin,
**characterized in that** the following formulations are excluded:
| | **B1** | **D1** | **E1** | **B2** | **D2** | **E2** | **F2** |
|---|---|---|---|---|---|---|---|
| **INCI** | **m [%]** | **m [%]** | **m [%]** | **m [%]** | **m [%]** | **m [%]** | **m [%]** |
| Hydroxypropyl starch phosphate | 0.5 | 1.5 | 0.75 | 0.5 | 0.7 | 1 | 2.5 |
| Coco-caprylate/caprate | | | 3 | | 3 | | |
| Coco-caprylate | | | | 4 | | | |
| Decyl cocoate | | | | | | | 3 |
| Simmondsia chinensis seed oil | | | 3 | | | | |
| Vegetable oil | | 4 | | | | | |
| Shea butter ethyl esters | | | | | | | 3 |
| Caprylic/capric triglycerides | | | | | | 3 | |
| Dicaprylyl ether | 4 | 2 | 1.5 | | 1.5 | | |
| Prunus amygdalus dulcis oil | | | | | | 2 | |
| Brassica campestris seed oil | 4 | | | | | | |
| Cocos nucifera oil | | | | | | | 0.5 |
| Coco-glycerides | | | | 6 | 3 | | |
| Theobroma cacao seed butter | | | 1 | | | | 1 |
| Sodium cetearyl sulfate | 0.15 | 0.1 | 0.15 | 0.15 | 0.15 | 0.15 | 0.2 |
| Sodium stearoyl glutamate | | | | | | | |
| Glyceryl stearate SE | 1.5 | 1.7 | 2.5 | 1.5 | 2 | 2 | 2.5 |
| Glyceryl stearate | 1 | | 0.5 | 1 | 1 | | 0.5 |
| Cetearyl alcohol | 4 | 1.2 | | 4 | 4 | 2 | |
| Stearyl alcohol | | | 3 | | | | |
| Butyrospermum parkii butter | 1 | 1 | | 1 | 1 | | |
| Cetyl palmitate | | | 0.5 | | | 1 | |
| Glycerol | 7 | 7 | 7 | 7 | 7 | 7 | 5 |
| Phenoxyethanol | 0.9 | | 0.9 | 0.9 | 0.5 | | |
| Ethylhexylglycerin | 0.2 | 0.2 | 0.2 | 0.2 | 0.5 | 0.3 | |
| Benzyl alcohol | | 0.2 | | | | 0.2 | |
| Polyglyceryl-2 caprate | | | | | | | 0.5 |
| Alcohol | | 4 | 4 | | 3 | | 4 |
| Perfume | | 0.2 | | | 0.4 | | 0.3 |
| Aqua | to 100 | to 100 | to 100 | to 100 | to 100 | to 100 | to 100 |

3. Emulsion according to Claim 1 or use according to Claim 2, **characterized in that** the alkali metal alkyl sulfates are selected from the group consisting of sodium cetyl sulfate, sodium stearyl sulfate and sodium cetearyl sulfate.

4. Emulsion or use according to any of the preceding claims, **characterized in that** the total proportion of alkali metal alkyl sulfates present is from 0.05% by weight to 2.0% by weight, more preferably from 0.08% by weight to 1.5% by weight and most preferably from 0.1% by weight to 0.3% by weight, based on the total weight of the emulsion.

5. Emulsion or use according to any of the preceding claims, **characterized in that** the proportion of hydroxypropyl starch phosphate is from 0.1% by weight to 5% by weight, preferably from 0.15% by weight to 4.0% by weight and particularly preferably from 0.2% by weight to 3% by weight, based on the total weight of the emulsion.

6. Emulsion or use according to any of the preceding claims, **characterized in that** the total proportion of glyceryl stearate SE in the emulsion is from 0.1% by weight to 6.0% by weight, preferably 0.5% by weight to 5.0% by weight and particularly preferably from 1.0% by weight to 3.0% by weight, wherein the figures refer to the total weight of the emulsion.

7. Emulsion or use according to any of the preceding claims, **characterized in that** the proportion of the oil phase of the emulsion is from more than 2% by weight to 30% by weight, preferably from more than 3% by weight to 20% by weight and particularly preferably from more than 4% by weight to 15.5% by weight, based on the total weight of the emulsion.

8. Emulsion or use according to any of the preceding claims, **characterized in that** the emulsion comprises glyceryl stearate, wherein the proportion of glyceryl stearate is preferably from 0.3% to 7% by weight, more preferably from 0.35% to 5% by weight and particularly preferably from 0.5% to 1.5% by weight, based on the total weight of the emulsion.

9. Emulsion or use according to any of the preceding claims, **characterized in that** the emulsion comprises one or more fatty alcohols having 14 to 22 carbon atoms, wherein the total proportion of said fatty alcohols having 14 to 22 carbon atoms is advantageously from 0.1% by weight to 7.0% by weight, preferably 0.5% by weight to 6.0% by weight and particularly preferably from 1% by weight to 5.0% by weight, based on the total weight of the emulsion.

10. Emulsion or use according to any of the preceding claims, **characterized in that** the O/W emulsion of the present invention does not contain any mineral oil.

11. Emulsion or use according to any of the preceding claims, **characterized in that** the emulsion comprises coco-glycerides, wherein the proportion of coco-glycerides is preferably from 0.5% to 9% by weight and particularly preferably from 1% to 8% by weight, based on the total weight of the emulsion.

12. Emulsion or use according to any of the preceding claims, **characterized in that** the emulsion comprises coco-caprylate/caprate, wherein the proportion of coco-caprylate/caprate is preferably from 0.5% to 7% by weight and particularly preferably from 2% to 6% by weight, based on the total weight of the emulsion.

13. Emulsion or use according to any of the preceding claims, **characterized in that** the emulsion comprises Butyrospermum parkii butter, wherein Butyrospermum parkii butter is present preferably at a proportion from 0.3% to 2% by weight and particularly preferably from 0.5% to 1.5% by weight, based on the total weight of the emulsion.

14. Emulsion or use according to any of the preceding claims, **characterized in that** the emulsion comprises further surfactants and/or emulsifiers only at a maximum proportion of 2% by weight, preferably at a maximum proportion of 1.5% by weight, wherein the figures refer to the total weight of the emulsion.

15. Emulsion or use according to any of the preceding claims, **characterized in that** the emulsions have a viscosity of 3000 mPa·s to 6000 mPa·s 24 hours after preparation at 25°C, wherein these figures refer to a measurement with a Rheomat R 123 from ProRheo using measurement bob 1 at a speed of 62.5 min⁻¹.

## Revendications

1. Émulsion H/E cosmétique, exempte d'acrylate et de silicone, contenant
a) au moins une substance choisie dans le groupe des alkylsulfates de métal alcalin, le groupe alkyle présentant à chaque fois 15 à 19 atomes de carbone,
b) du phosphate d'hydroxypropylamidon et
c) du stéarate de glycéryle SE,
**caractérisée en ce que** les formulations suivantes sont exclues :
| | **B1** | **D1** | **E1** | **B2** | **D2** | **E2** | **F2** |
|---|---|---|---|---|---|---|---|
| **INCI** | **m[%]** | **m[%]** | **m[%]** | **m[%]** | **m[%]** | **m[%]** | **m[%]** |
| Phosphate d'hydroxypropylamidon | 0,5 | 1,5 | 0,75 | 0,5 | 0,7 | 1 | 2,5 |
| Caprylate/Caprate de coco | | | 3 | | 3 | | |
| Caprylate de coco | | | | 4 | | | |
| Cocoate de décyle | | | | | | | 3 |
| Huile de germes de Simmondsia Chinensis | | | 3 | | | | |
| Huile végétale | | 4 | | | | | |
| Ester éthylique de beurre de karité | | | | | | | 3 |
| Triglycéride caprylique/caprique | | | | | | 3 | |
| Dicaprylyléther | 4 | 2 | 1,5 | | 1,5 | | |
| Huile de Prunus Amygdalus Dulcis | | | | | | 2 | |
| Huile de germes de Brassica Campestris | 4 | | | | | | |
| Huile de coco | | | | | | | 0,5 |
| Glycérides de coco | | | | 6 | 3 | | |
| Beurre de germes de cacao de théobromoa | | | 1 | | | | 1 |
| Cétéarylsulfate de sodium | 0,15 | 0,1 | 0,15 | 0,15 | 0,15 | 0,15 | 0, 2 |
| Stéaroylglutamate sodique | | | | | | | |
| Stéarate de glycéryle SE | 1,5 | 1,7 | 2,5 | 1,5 | 2 | 2 | 2,5 |
| Stéarate de glycéryle | 1 | | 0,5 | 1 | 1 | | 0,5 |
| Alcool cétéarylique | 4 | 1,2 | | 4 | 4 | 2 | |
| Alcool stéarylique | | | 3 | | | | |
| Beurre de karité | 1 | 1 | | 1 | 1 | | |
| Palmitate de cétyle | | | 0,5 | | | 1 | |
| Glycérol | 7 | 7 | 7 | 7 | 7 | 7 | 5 |
| Phénoxyéthanol | 0,9 | | 0,9 | 0,9 | 0,5 | | |
| Éthylhexylglycérol | 0,2 | 0,2 | 0,2 | 0,2 | 0,5 | 0,3 | |
| Alcool benzylique | | 0,2 | | | | 0,2 | |
| Caprate de polyglycéryle-2 | | | | | | | 0,5 |
| Alcool | | 4 | 4 | | 3 | | 4 |
| Parfums | | 0,2 | | | 0,4 | | 0,3 |
| Eau | Ad 100 | Ad 100 | Ad 100 | Ad 100 | Ad 100 | Ad 100 | Ad 100 |

2. Utilisation de mélange constitué par a) au moins une substance choisie dans le groupe des alkylsulfates de métal alcalin, le groupe alkyle présentant à chaque fois 15 à 19 atomes de carbone,
b) du phosphate d'hydroxypropylamidon et
c) du stéarate de glycéryle SE,
dans une émulsion H/E cosmétique, exempte d'acrylate et de silicone pour la réduction de résidus blancs lors de l'application de l'émulsion sur la peau,
**caractérisée en ce que** les formulations suivantes sont exclues :
| | **B1** | **D1** | **E1** | **B2** | **D2** | **E2** | **F2** |
|---|---|---|---|---|---|---|---|
| **INCI** | **m[%]** | **m[%]** | **m[%]** | **m[%]** | **m[%]** | **m[%]** | **m[%]** |
| Phosphate d'hydroxypropylamidon | 0,5 | 1,5 | 0,75 | 0,5 | 0,7 | 1 | 2,5 |
| Caprylate/Caprate de coco | | | 3 | | 3 | | |
| Caprylate de coco | | | | 4 | | | |
| Cocoate de décyle | | | | | | | 3 |
| Huile de germes de Simmondsia Chinensis | | | 3 | | | | |
| Huile végétale | | 4 | | | | | |
| Ester éthylique de beurre de karité | | | | | | | 3 |
| Triglycéride caprylique/caprique | | | | | | 3 | |
| Dicaprylyléther | 4 | 2 | 1,5 | | 1,5 | | |
| Huile de Prunus Amygdalus Dulcis | | | | | | 2 | |
| Huile de germes de Brassica Campestris | 4 | | | | | | |
| Huile de coco | | | | | | | 0,5 |
| Glycérides de coco | | | | 6 | 3 | | |
| Beurre de germes de cacao de théobromoa | | | 1 | | | | 1 |
| Cétéarylsulfate de sodium | 0,15 | 0,1 | 0,15 | 0,15 | 0,15 | 0,15 | 0, 2 |
| Stéaroylglutamate sodique | | | | | | | |
| Stéarate de glycéryle SE | 1,5 | 1,7 | 2,5 | 1,5 | 2 | 2 | 2,5 |
| Stéarate de glycéryle | 1 | | 0,5 | 1 | 1 | | 0,5 |
| Alcool cétéarylique | 4 | 1,2 | | 4 | 4 | 2 | |
| Alcool stéarylique | | | 3 | | | | |
| Beurre de karité | 1 | 1 | | 1 | 1 | | |
| Palmitate de cétyle | | | 0,5 | | | 1 | |
| Glycérol | 7 | 7 | 7 | 7 | 7 | 7 | 5 |
| Phénoxyéthanol | 0,9 | | 0,9 | 0,9 | 0,5 | | |
| Éthylhexylglycérol | 0,2 | 0,2 | 0,2 | 0,2 | 0,5 | 0,3 | |
| Alcool benzylique | | 0,2 | | | | 0,2 | |
| Caprate de polyglycéryle-2 | | | | | | | 0,5 |
| Alcool | | 4 | 4 | | 3 | | 4 |
| Parfums | | 0,2 | | | 0,4 | | 0,3 |
| Eau | Ad 100 | Ad 100 | Ad 100 | Ad 100 | Ad 100 | Ad 100 | Ad 100 |

3. Émulsion selon la revendication 1 ou utilisation selon la revendication 2, **caractérisée en ce que** les alkylsulfates de métal alcalin sont choisis dans le groupe acétylsulfate de sodium, stéarylsulfate de sodium ou cétéarylsulfate de sodium.

4. Émulsion ou utilisation selon l'une des revendications précédentes, **caractérisée en ce que** la proportion totale des alkylsulfates de métal alcalin est de 0,05% en poids à 2,0% en poids, plus préférablement de 0,08% en poids à 1,5% en poids et le plus préférablement de 0,1% en poids à 0,3% en poids, par rapport au poids total de l'émulsion.

5. Émulsion ou utilisation selon l'une des revendications précédentes, **caractérisée en ce que** la proportion de phosphate d'hydroxypropylamidon est de 0,1% en poids à 5% en poids, de préférence de 0,15% en poids à 4,0% en poids et en particulier de préférence de 0,2% en poids à 3% en poids, par rapport au poids total de l'émulsion.

6. Émulsion ou utilisation selon l'une des revendications précédentes, **caractérisée en ce que** la proportion totale de stéarate de glycérol SE dans l'émulsion est de 0,1% en poids à 6,0% en poids, de préférence de 0,5% en poids à 5,0% en poids et en particulier de préférence de 1,0% en poids à 3,0% en poids, les indications se rapportant au poids total de l'émulsion.

7. Émulsion ou utilisation selon l'une des revendications précédentes, **caractérisée en ce que** la proportion de la phase huileuse de l'émulsion est de plus de 2% en poids à 30% en poids, de préférence de plus de 3% en poids à 20% en poids et en particulier de préférence de plus de 4% en poids à 15,5% en poids, par rapport au poids total de l'émulsion.

8. Émulsion ou utilisation selon l'une des revendications précédentes, **caractérisée en ce que** l'émulsion contient du stéarate de glycéryle, la proportion de stéarate de glycéryle étant de préférence de 0,3 à 7% en poids, plus préférablement de 0,35 à 5% en poids et en particulier de préférence de 0,5 à 1,5% en poids, par rapport au poids total de l'émulsion.

9. Émulsion ou utilisation selon l'une des revendications précédentes, **caractérisée en ce que** l'émulsion contient un ou plusieurs alcools gras comprenant 14 à 22 atomes de carbone, la proportion totale de ces alcools gras comprenant 14 à 22 atomes de carbone étant avantageusement de 0,1% en poids à 7,0% en poids, de préférence de 0,5% en poids à 6,0% en poids et en particulier de préférence de 1% en poids à 5,0% en poids, par rapport au poids total de l'émulsion.

10. Émulsion ou utilisation selon l'une des revendications précédentes, **caractérisée en ce que** l'émulsion H/E de la présente invention ne contient pas d'huile minérale.

11. Émulsion ou utilisation selon l'une des revendications précédentes, **caractérisée en ce que** l'émulsion contient des glycérides de coco, la proportion de glycérides de coco étant de préférence de 0,5 à 9% en poids et en particulier de préférence de 1 à 8% en poids, par rapport au poids total de l'émulsion.

12. Émulsion ou utilisation selon l'une des revendications précédentes, **caractérisée en ce que** l'émulsion contient des caprylates/caprates de coco, la proportion de caprylates/caprates de coco étant de préférence de 0,5 à 7% en poids et en particulier de préférence de 2 à 6% en poids, par rapport au poids total de l'émulsion.

13. Émulsion ou utilisation selon l'une des revendications précédentes, **caractérisée en ce que** l'émulsion contient du beurre de karité, le beurre de karité étant de préférence contenu en une proportion de 0,3 à 2% en poids et en particulier de préférence de 0,5 à 1,5% en poids par rapport au poids total de l'émulsion.

14. Émulsion ou utilisation selon l'une des revendications précédentes, **caractérisée en ce que** l'émulsion ne contient d'autres tensioactifs et/ou émulsifiants qu'en une proportion maximale de 2% en poids, de préférence en une proportion maximale de 1,5% en poids, les indications se rapportant au poids total de l'émulsion.

15. Émulsion ou utilisation selon l'une des revendications précédentes, **caractérisée en ce que** les émulsions présentent, 24 heures après la préparation, à 25°C, une viscosité de 3000 mPa.s à 6000 mPa.s, ces indications se rapportant à une mesure à l'aide d'un Rheomat R 123 de la société ProRheo avec le corps de mesure 1 à une vitesse de rotation de 62,5 min⁻¹.
